Europäisches Patentamt

European Patent Office

Office européen des brevets

(11) Publication number: **0 420 996 A1**

## EUROPEAN PATENT APPLICATION
### published in accordance with Art. 158(3) EPC

(21) Application number: 90906431.3

(22) Date of filing: 13.03.90

(86) International application number:
PCT/SU90/00068

(87) International publication number:
WO 90/11794 (18.10.90 90/24)

(51) Int. Cl.5: **A61M 27/00**

(30) Priority: 10.04.89 SU 4675188

(43) Date of publication of application:
**10.04.91 Bulletin 91/15**

(84) Designated Contracting States:
**BE CH DE FR GB IT LI SE**

(71) Applicant: POLTAVSKY MEDITSINSKY
STOMATOLOGICHESKY INSTITUT
ul. Shevchenko, 23,
Poltava 314000(SU)

(72) Inventor: MAZURIK, Sergei Mikhailovich
ul. Lenina, 92-57
Poltava, 314022(SU)
Inventor: SOKOLOV, Andrei Nikolaevich
ul. 60 let SSSR, 3-20
Poltava, 314022(SU)

(74) Representative: Lerwill, John et al
A.A. Thornton & Co. Northumberland House
303-306 High Holborn
London, WC1V 7LE(GB)

(54) T-SHAPED DRAINAGE TUBE.

(57) The invention relates to medical techniques. The drainage tube consists of a longitudinal part (1) and a transversal part (2) interconnected by means of a detachable connection, for example, a screw joint. The T-shaped drainage tube is intended to be used in hospitals which perform surgery of biliary ducts.

FIG.2

EP 0 420 996 A1

## T-DRAIN TUBE

Technical Field

The invention relates generally to medical engineering, more specifically to T-drain tubes applied for drainage of the common bile duct.

Background Art

One prior-art T-drain tube is known to be made of an elastic material (latex) and be used for transpapillary drainage of the common bile duct (cf. T-Drain, Cattell, code No. 2575, product of the USA available from Inmed Corporation (2950, Pacific Drive, Norcross, Ga 30071).

The heretofore-known drain tube is composed of a longitudinal and transverse member made as an integral unit. In the course of surgery, the transverse member of said drain tube is placed in the common bile duct, while the longitudinal member thereof is brought outside through a special incision. One of the ends of the transverse member of the tube is passed through the major diodenal pailla into the duodenum to establish unobstructed bile discharge. When withdrawing the drain tube from the common bile duct a double-lumen transverse member is passed through its opening established round a single-lumen longitudinal member of the drain tube. As a result, the walls of the common bile duct are lacerated and a pronounced traumatic injury to the surrounding soft tissues ensues. Moreover, it is through the gaping wound of the common bile duct that a great amount of bile is free to discharge after withdrawal of the drain tube, which might be causative of diverse complications. In this respect, surgeons are to place limitation upon indications for drainage of the common bile duct, using the known T-drain tube, which is in fact the optimum drainage system of the extrahepatic bile ducts.

Summary of the Invention

It is a primary and essential object of the invention to provide a T-drain tube of such a construction that would prevent any injury to the walls of the common bile duct and to the surrounding tissues when being withdrawn from the common bile duct in the postoperative period.

Said object is accomplished due to the fact that in a T-drain tube having a longitudinal member and a transverse member, according to the invention, the longitudinal and transverse members are interconnected through a separable joint.

The T-drain tube, according to the present invention, prevents traumatic lesion of the walls of the common bile duct and of the surrounding soft tissues when being withdrawn from the common bile duct within the postoperative period. Expenses for the manufacture of T-drain tubes, according to the invention, is but slightly in excess of those for production of the heretofore-known drain tubes.

Brief Description of the Drawings

In what follows the invention is illustrated by a detailed description of a specific exemplary embodiment thereof with reference to the accompanying drawings, wherein:

FIG. 1 is a longitudinal sectional view of a T-drain tube, according to the invention;

FIG. 2 is an isometric view of FIG. 1 showing the longitudinal and transverse members of the drain tube disengaged; and

FIG. 3 is an arrangement diagram of the T-drain tube, according to the invention, in the common bile duct before withdrawing the drain tube within the postoperative period.

Best Mode of Carrying Out the Invention

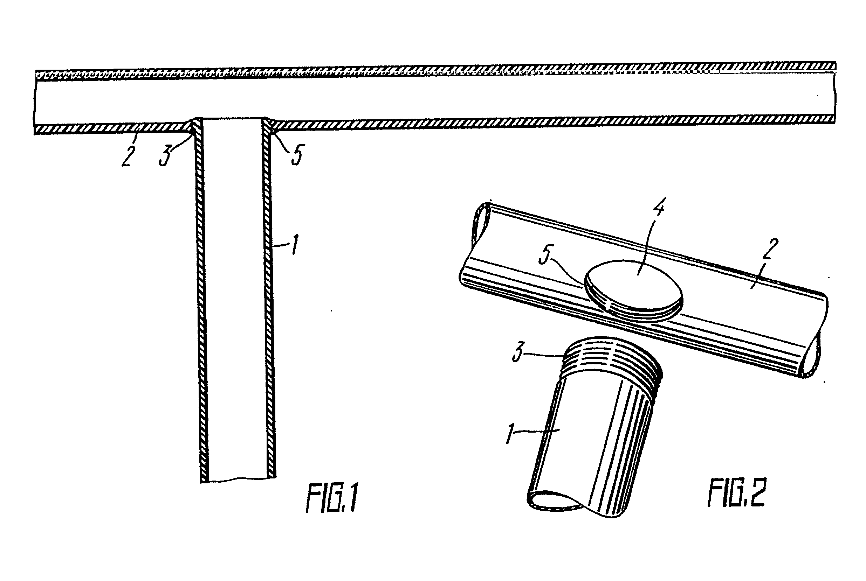

The T-drain tube presented in FIGS 1, 2 comprises a longitudinal member 1 and a transverse member 2, which are interconnected through a separable joint, e,g., a threaded joint having a small thread pitch (FIG. 2). The longitudinal member 1 is shaped as a single-lumen tube one of whose ends is provided with a male thread. The transverse member 2 is also shaped as a single-lumen tube whose wall has an opening 4 provided with a female thread 5 engageable with the thread 3 as a screw and a nut. Used to food advantage as a separable joint of the longitudinal member 1 and the transverse member 2 of the drain tube, according to the invention, can be also a bayonet joint (omitted in the Drawing).

The T-drain tube, according to the invention, is applied as follows.

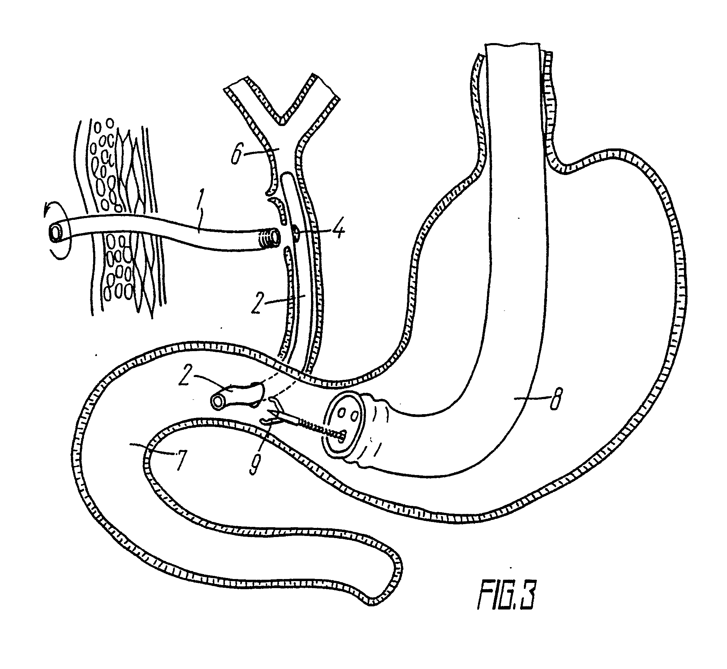

Prior to installing the drain tube its longitudinal member 1 is held to the transverse member 2 by making two or three revolutions in the threaded joint therebetween. During surgery the transverse member 2 of the T-drain tube is introduced into the common bile duct 6 (FIG. 3) through the choledochotomy opening in such a way that the distal end of the transverse member 2 is passed through the major duodenal pailla into the duodenum 7, The longitudinal member 1 is brought outwards through a counter-opening in the right hypochondrium. To withdraw the drain tube within the postoperative period, the longitudinal member 1 is given two or three backward revolutions (with reference to its direction of rotation before surgery), with the result that the longitudinal member 1 is disengaged from the transverse member 2 and is withdrawn outwards as a standard single-lumen tube. Then the major duodenal pailla with the end of the transverse member 2 extending therefrom is located visually in the lumen of the duodenum 7 with the aid of a gastroduodenosoope 8. Next the end of the transverse member 2 is gripped with the aid of a forceps 9 that has been passed through the instrumental canal of the gastroduodenoscope 8 and the latter is withdrawn together with the transverse member 2 of the drain tube. Thus, any traumatic lesion of the walls of the common bile duct 6 and of the soft tissues is prevented when withdrawing the T-drain tube for transpapillary drainage within the postoperative period.

According to one of the versions of practical application of the drain tube, according to the invention, the transverse member 2 may be pulled out but partially of the common bile duct 6 into the lumen of the duodenum 8 using the forceps 9. As a result the opening 4 will be displaced with respect to the wound opening of the common bile duct 6 so that bile will not escape from the wound into the abdominal cavity, thus preventing bile from flowing out of the wound of the common bile duct 6 into the abdominal cavity just after withdrawing the longitudinal meter 1 of the drain tube, The transverse member 2 of the drain tube is withdrawn in a few days with the aid of a duodenoscope.

Industrial Applicability

The invention can find application in medical institutions engaged in surgery of the bile ducts.

Claims

1. A T-drain tube having a longitudinal member (1) and a transverse member (2), CHARACTERIZED in that the longitudinal and transverse members (1 and 2) are interconnected through a separable joint.

FIG.1

FIG.2

EP 0 420 996 A1

FIG.3

# INTERNATIONAL SEARCH REPORT

International Application No PCT/SU90/00068

## I. CLASSIFICATION OF SUBJECT MATTER (If several classification symbols apply, indicate all)

According to International Patent Classification (IPC) or to both National Classification and IPC

IPC⁵: A61M 27/00

## II. FIELDS SEARCHED

| Minimum Documentation Searched | |
|---|---|
| Classification System | Classification Symbols |
| IPC⁴ | A61M 27/00 |

Documentation Searched other than Minimum Documentation
to the Extent that such Documents are Included in the Fields Searched

## III. DOCUMENTS CONSIDERED TO BE RELEVANT

| Category* | Citation of Document, with indication, where appropriate, of the relevant passages | Relevant to Claim No. |
|---|---|---|
| A | SU, AI, 1410988 (DNEPROPETROVSKY OBLASTNOI OTDEL ZDRAVOOKHRANENIA), 23 July 1988 (23.07.88), the claims, figure 2 | 1 |
| A | KATALOG CATALOGUE-CATALOGO N15, 1966, Injections-spritzen und kanülen-fabrik, Apparate-Bau, Diagnostische instrumente CTP.17 KPOMATA 12230-12236 (DE) | 1 |
| A | KATALOG, WILLY RUSCH Rommelshausen bei Stuttgart, 1970, part III/2 Gastroenterologie Chirurgie Drainagen, 423601 (DE) | 1 |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 26 June 1990 (26.06.90) | 17 July 1990 (17.07.90) |
| International Searching Authority | Signature of Authorized Officer |
| ISA/SU | |

Form PCT/ISA/210 (second sheet) (January 1985)